# EUROPEAN PATENT APPLICATION

(11) **EP 2 511 529 A1**
(43) Date of publication of application: **17.10.2012**
(21) Application number: 11382112.8
(22) Date of filing: 15.04.2011
(51) Int. Cl.: F04B 43/02, A61M 1/10, F04B 43/04, F04B 53/10, A61M 5/142

(54) **Impulsion core for a fluid micropump**

(71) Applicant: Ikerlan, S. Coop., 20500 Arrasate-Mondragon (ES); Fundació Privada Institut de Recerca Biomèdica, 08028 Barcelona (ES); Fundació Privada Institució Catalana De Recerca I Estudis Avançants, 08010 Barcelona (ES)
(72) Inventor: EZKERRA FERNÁNDEZ, Aitor, E-48920, Portugalete (Bizkaia) (ES); BERGANZO RUIZ, Javier, E-01010, Vitoria-Gasteiz (Araba) (ES); RIBAS DE POUPLANA, Lluis, E-08010, Barcelona (ES); CORTÉS I CLOSAS, Alfred, E-08041, Barcelona (ES)
(74) Representative: ABG Patentes, S.L.

(57) **Abstract**

The present invention relates to an impulsion core (1) for a fluid micropump comprising a plate (2) with a first cavity (3) and second (4) and third (5) cavities communicated with the first cavity (3), the second cavity (4) and third cavity (5) having at least one one-way valve (6, 8) with a support (6.1, 8.1) and a cantilever (6.2, 8.2) emerging from the support (6.1, 8.1), the cantilever (6.2, 8.2) comprising a first sector (6.2.1, 8.2.1) for closing a first opening (7, 9) and a second sector (6.2.2, 8.2.2) arranged between the support (6.1, 8.1) and the first sector (6.2.1, 8.2.1), one of the one-way valves (6, 8) allowing the entrance of fluid into the first cavity (3) and the other one-way valve allowing the exit of fluid from the first cavity (3), the impulsion core (1) comprising a fluid inlet (10) and a fluid outlet (11) and a flexible membrane (14) closing the first cavity (3), to impulse a fluid by means of applying a pressing force on said membrane (14), the support (6.1, 8.1) and the second sector (6.2.2, 8.2.2) of the cantilever (6.2, 8.2) being separated from the walls of the cavity (4, 5) in which the opening (7, 9) is located, establishing a communication channel through which a fluid can circulate between either side of the cantilever (6.2, 8.2) around the support (6.1, 8.1).

## Description

### Technical Field of the Invention

The present invention is comprised in the field of miniaturized systems for modeling a continuous flow, preferably for blood circulation, and it relates to an impulsion core for a fluid micropump.

### Background of the Invention

Miniaturized systems for modeling blood circulation based on Lab-on-a-chip (LoC) technologies have a wide range of applications in clotting studies, tumor cell detection in blood and parasitology, among others. These systems are relevant due to the possibility of obtaining ex-vivo information, overcoming the possibilities of current methods.

Blood transport systems, such as those based on electrolysis, traveling-wave dielectrophoresis, piezoelectric, thermal, impulsion, colloidal, thermopneumatic or viscous systems, are known. The main purpose of these systems is to prevent cell damage which, in the case of erythrocytes, involves membrane damage and the release of the hemoglobin content, which is known as hemolysis.

Document US2008161779, for example, describes a nano pump comprising a cavity, a membrane for pumping, an inlet channel and an outlet channel, and an inlet valve and an outlet valve comprising cantilevers, each associated with its channel corresponding.

In most cases, the flow rate and the speeds provided by blood transport systems are very low, which favors cell damage not occurring. However, the speeds at which these systems work are insufficient in preventing sedimentation when circulation and not transport is to be modeled. Furthermore, transport differs from circulation not only in the range of required speeds, but also in the exposure of erythrocytes to shearing stress. Therefore, while in systems designed for transport the erythrocytes can remain unruptured under rupture stress levels for a certain time, in a circulation system the erythrocytes can be subjected to constant stress for hours, so the design of the system and the selection of materials must be done more carefully.

A miniaturized pump which allows the circulation of the fluid at a high enough speed to prevent sedimentation, at the same time minimizing cell damage, is therefore necessary.

### Summary of the Invention

The present invention provides a solution to the problems described above by means of an impulsion core for a fluid micropump according to claim 1. The dependent claims define preferred embodiments of the invention.

Micropump can typically be understood as those pumps with flow rates of about 16 ml/min or less.

The impulsion core according to the invention comprises a plate with a first cavity formed therein and a second cavity and a third cavity, both communicated with the first cavity, and the second cavity and the third cavity being provided with at least one one-way valve each, formed by means of a support and a cantilever emerging from the support and it is configured to act by bending and establish the closure of valve, one of the one-way valves allowing the entrance of fluid into the first cavity and the other one-way valve allowing the exit of fluid from the first cavity in an operative mode. The at least one cantilever comprises a first sector suitable for closing an opening in a valve closed position and a second sector which is arranged between the support and the first sector. The impulsion core additionally comprises a fluid inlet from the outside of the plate towards the third cavity, and a fluid outlet from the second cavity towards the outside of the plate. A flexible membrane closes the first cavity and is suitable for the impulsion of a fluid by means of applying a pressing force on said membrane in an operative situation. The impulsion core is characterized in that the support and the second sector of the cantilever are separated from the walls of the cavity in which the opening which closes the valve is located, such that a communication channel through which a fluid can circulate between either side of the cantilever around the support is established.

The use of cantilevers as one-way valves minimally affects the impulsion core, because they do not collapse, they do not adhere to the valve seat and they do not bend towards the inside of the channel during operation, as occurs with other types of valves.

Furthermore, since the second sector of the cantilever and the support are separated from the walls of the cavity in which the cantilever is located, such that a fluid communication channel between either side of the cantilever around the support is established, it allows the fluid and the bubbles to be able to flow around the cantilever, eliminating dead spaces, reducing the obstacles imposed by the presence of the structures and eliminating recirculation regions. Particles present in the fluid are thereby prevented from being able to accumulate around the root of the cantilever, which would cause the sedimentation of such particles taking place and preventing the proper operation of the valve.

In the event that the fluid is blood, the effect of preventing the sedimentation of particles around the valve is particularly advantageous because the erythrocyte accumulation in backwash areas and around obstacles increases the stress to which they are subjected and favors the rupture thereof, causing unwanted hemolysis.

Advantageously, the impulsion core of the invention can be manufactured by means of a fast and simple manufacturing process because it can be performed in a single photolithography step, eliminating manual assembly problems and assuring process repeatability.

In a preferred embodiment of the impulsion core, the valve seat for supporting at least one of the cantilevers for closing the corresponding opening has two seats that are concave-curved towards the cavity in the allowed flow direction. In an even more preferred embodiment of the impulsion core, the first sector of at least one of the cantilevers further has a double curved sector in correspondence with the double valve seat.

In the systems of the state of the art, seepages into the valves leads to induced backflow which manifests as a backwards displacement of the fluid sample during the filling cycle. In the event of blood, the induced backflow is one of the effects contributing to hemolysis because the transition of a positive displacement to a negative displacement worsens the stress levels of the erythrocytes. With the valve seat according to a preferred embodiment of the invention, the closure of the valve is improved, minimizing the risk of leaks and seepages.

In a particular embodiment of the invention, the impulsion core is manufactured in SU-8. This material is advantageous due to its low roughness, which is in the nanometric order.

In a preferred embodiment of the impulsion core according to the invention, the portion of membrane suspended on the first cavity comprises a reinforcing disc, preferably manufactured in SU-8, embedded therein. It is thereby assured that the portion of membrane on the first cavity remains substantially planar throughout its entire motion range, thus increasing the stroke volume and reducing the necessary actuation pressure.

In a preferred embodiment, the impulsion core comprises a stopper for limiting the motion range of the cantilever. The stopper assures that in a valve open position the cantilever remains within the elastic limit, which prolongs the cyclic life of the impulsion core.

In a preferred embodiment, the impulsion core comprises at least one support in the first cavity to prevent the membrane from knocking against the bottom of the cavity. In a fluid with particles in suspension, the crushing of the particles between the membrane and the bottom of the first cavity is thereby avoided. When the fluid is blood, avoiding the crushing of the erythrocytes is particularly advantageous, since it is a cause of hemolysis. Additionally or alternatively, the bottom of the first cavity can be concave enough to prevent the membrane from knocking against the bottom of the cavity, achieving a similar effect.

A mode of using the core according to the invention gives rise to a fluid micropump comprising an impulsion core according to the invention, retaining means for retaining the impulsion core, application means for applying a pressing force, configured to act on the membrane of the impulsion core, fluid connection means suitable for connecting with the fluid inlet of the impulsion core, and fluid connection means suitable for connecting with the fluid outlet of the impulsion core.

In a preferred embodiment, the retaining means for retaining the impulsion core are arranged in a first part of a casing, the application means for applying a pressing force are arranged in a second part of the casing, and there are fixing means for fixing the first part with respect to the second part. The first and second parts can be separated parts or they can be connected by means of a hinged attachment, which allows opening and closing the micropump like a book.

The fluid micropump according to the invention is self-priming and tolerates the presence of bubbles, it presents good tolerance to particles, without sedimentation thereof nor adhesion to the valve seat, and without a degraded operation.

In a second inventive aspect, the use of an impulsion core according to the first inventive aspect for the study of biological processes under closed circuit, continuous flow conditions is defined.

In a preferred embodiment, the invention relates to the use of the impulsion core according to the first inventive aspect for the study of biological processes associated with the malaria parasite *Plasmodium falciparum,* preferably for the study of erythrocyte invasion, cytoadherence and/or rosette formation, as well as the interactions between these biological processes.

One aspect of the invention comprises coating the walls of a closed circuit in which the induced flow is impulsed by the impulsion core according to the invention with endothelial receptors to simulate cytoadherence of erythrocytes infected by *P. falciparum.* Another aspect of the invention makes use of strains capable of inducing the erythrocytes to form rosettes in a closed circuit in which the induced flow is impulsed by the impulsion core according to the invention, in order to study how the cytoadherence and the presence of the rosettes affect the erythrocyte invasion in flow conditions. Since traditional assays are static, they cannot detect the possible interaction between cytoadherence and erythrocyte invasion.

In a preferred embodiment, the invention relates to the use of the impulsion core according to the first inventive aspect for the diagnosis of blood clotting disorders.

In a preferred embodiment, the invention relates to the use of the impulsion core according to the first inventive aspect for tumor cell detection in a biological fluid, preferably blood.

In a preferred embodiment, the invention relates to the use of the impulsion core according to the first inventive aspect for the study of biological processes and/or diseases which are critically affected by the flow and which would benefit from using a flow in a closed circuit in conditions that are favorable for the cells, such as clotting, hemophilia, thrombosis, platelet biology, circulating tumor cell (CTC) biology, tumor cell adhesion or leukocyte adhesion.

In a preferred embodiment, the invention relates to the use of the impulsion core according to the first inventive aspect for the clinical research of materials for stents or pharmacological research, preferably research of anti-clotting drugs or drug tests in relation to side effects on clotting.

All the features described in this specification (including the claims, description and drawings) can be combined in any combination, with the exception of combinations of such mutually exclusive features.

### Brief Description of the Drawings

To complement the description provided below and for the purpose of aiding to better understand the features of the invention a set of drawings is attached to the present specification in which the following has been depicted with an illustrative and non-limiting character:
Figure 1 shows an impulsion core according to an embodiment of the invention.
Figure 2 shows an enlarged detail of the impulsion core of Figure 1.
Figure 3 shows an impulsion core according to a second embodiment of the invention.
Figure 4 shows a schematic depiction of an impulsion core according to an embodiment of the invention.
Figure 5 shows a detail of a cantilever of an impulsion core according to an embodiment of the invention.
Figure 6 shows a micropump according to an embodiment of the invention.

### Embodiments of the Invention

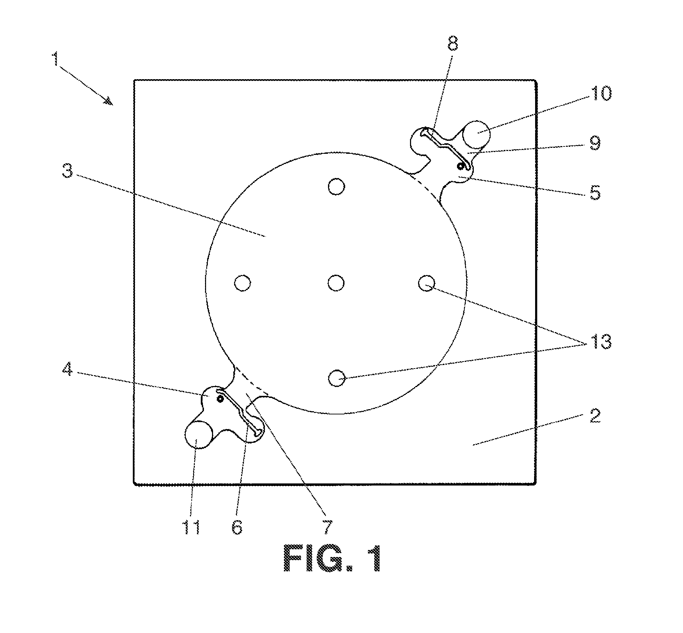

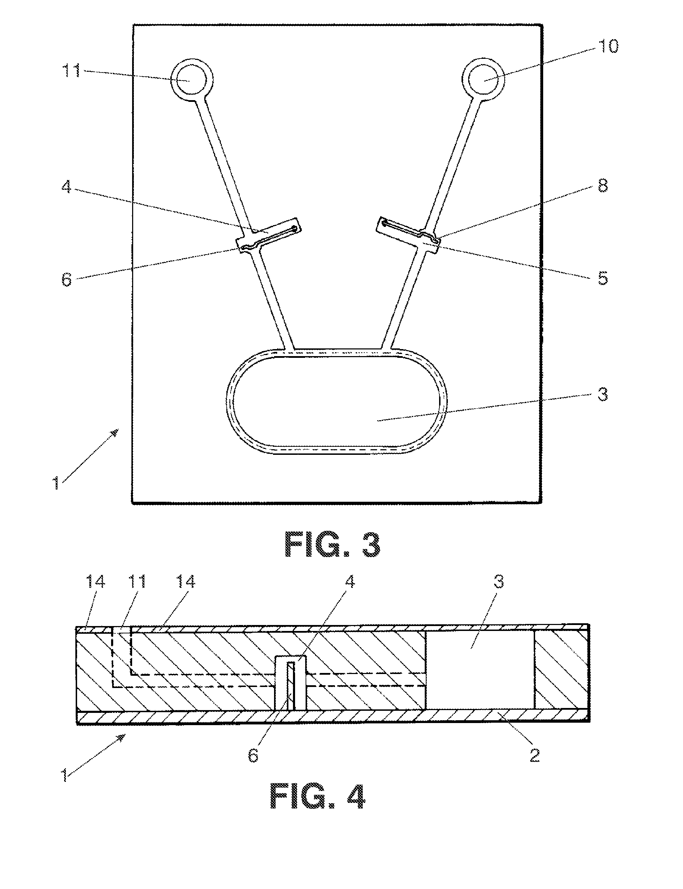

Figure 1 depicts an impulsion core (1) according to an embodiment of the invention. It has a plate (2) with a first central cavity (3) and two smaller additional cavities (4, 5) communicated with the first cavity (3) by means of respective openings. The cavities (3, 4, 5) are such that they do not perforate the plate (2) and they have a base. The second cavity (4) and the third cavity (5) have respective one-way valves (6, 8) formed by means of a support (6.1, 8.1) and a cantilever (6.2, 8.2). In the impulsion core (1) of the figure, the one-way valve (6) of the second cavity (4) is arranged such that it allows the exit of fluid from the first cavity (3) in an operative mode, whereas the one-way valve (8) of the second cavity (5) is arranged such that it allows the entrance of fluid into the first cavity (3) in a operative mode. It further has a fluid outlet (11) from the second cavity (4) towards the outside of the plate (2) and a fluid inlet (10) from the outside of the plate (2) towards the third cavity (5), both the inlet (10) and the outlet (11) being materialized in a capillary sector and a fluid inlet/outlet port. A flexible membrane (14), schematically depicted in Figure 4, completely covering the plate (2) except for the fluid inlet/outlet ports, closes the structure of cavities. The membrane (14) is suitable for receiving a pressing force in the portion covering the first cavity (3) and transmitting said pressing force to a fluid sample that is contained in the first cavity (3) during the operation of the impulsion core (1), causing its impulsion. The operating principle of the impulsion core (1) is based on the synchronous movement of the pair of cantilevers (6.2, 8.2) and the membrane (14). The pneumatic actuation of the membrane (14) operates an alternating opening/closing movement in the pair of one-way valves (6, 8), producing a one-way flow.

The membrane (14) can be manufactured in elastomer, such as PDMS (polydimethylsiloxane). If a disc of a more rigid material embedded in the portion of membrane suspended on the first cavity (3) is provided, the performance of the membrane (14) improves as it remains planar throughout its entire motion range, thereby increasing the stroke volume and reducing the required actuation pressure. Said more rigid material can be SU-8. SU-8, also known as Epon SU8, is a high-resolution epoxy-based negative photoresist with a certain degree of biocompatibility.

Figure 1 further shows five supports (13) arranged in the first cavity (3) to prevent the membrane (14) from being able to hit against the bottom of the first cavity (3) in its pneumatic movement.

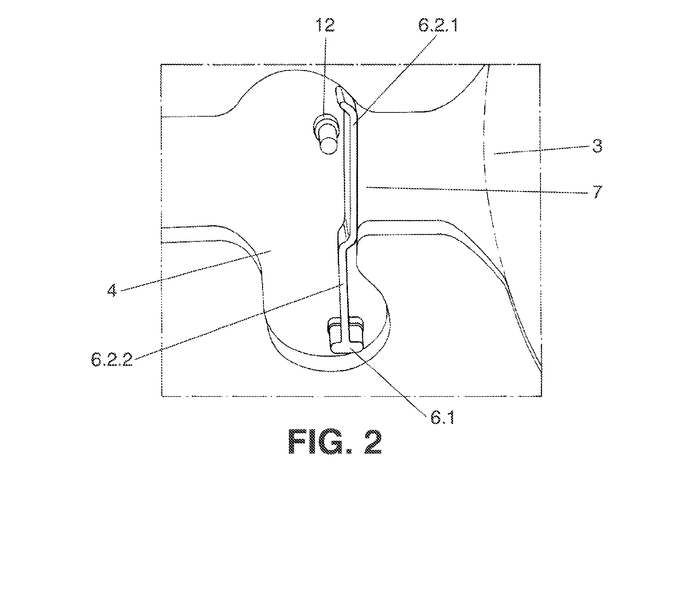

An enlarged view of the second cavity (4) is observed in Figure 2. In this figure it can be seen that the support (6.1) and the second sector (6.2.2) of the cantilever (6.2) arranged in the second cavity (4) are spaced from the walls of the cavity (4) in which the opening (7) closed by the cantilever (6.2) in the valve closed position is located. A fluid communication channel which allows the circulation of fluid between either side of the cantilever (6.2) around the support (6.1) is thus established. In this embodiment, the second cavity (4) and the third cavity (5) are bean-shaped, the contour of which is suitable for minimizing the accumulation of particles in the walls.

The arrangement of cavities (3, 4, 5), their sizes and the communication between them can be designed according to needs, the performance of the impulsion core being otherwise analogous. Figure 3 shows an impulsion core (1) according to an embodiment of the invention, having a plate (2) with a first elongated cavity (3) and the second elongated cavity (4) and third elongated cavity (5) smaller than the first cavity (3). The fluid communication between the first cavity (3) and the second cavity (4) and third cavity (5) is carried out through respective channels, which are comparatively longer than the communication openings observed in the embodiment of Figure 1.

Figure 4 shows a cross-section of a schematic depiction of an impulsion core according to the invention. In this figure, the plate (2) with the first cavity (3) and the second cavity (4) depicted therein, a communication channel between both depicted with a dotted line, a fluid outlet (11) from the second chamber (4) to the outside and a membrane (14) completely covering the plate with the exception of the fluid inlet/outlet ports, can be seen. A cantilever (6.2) is depicted in the second cavity (4).

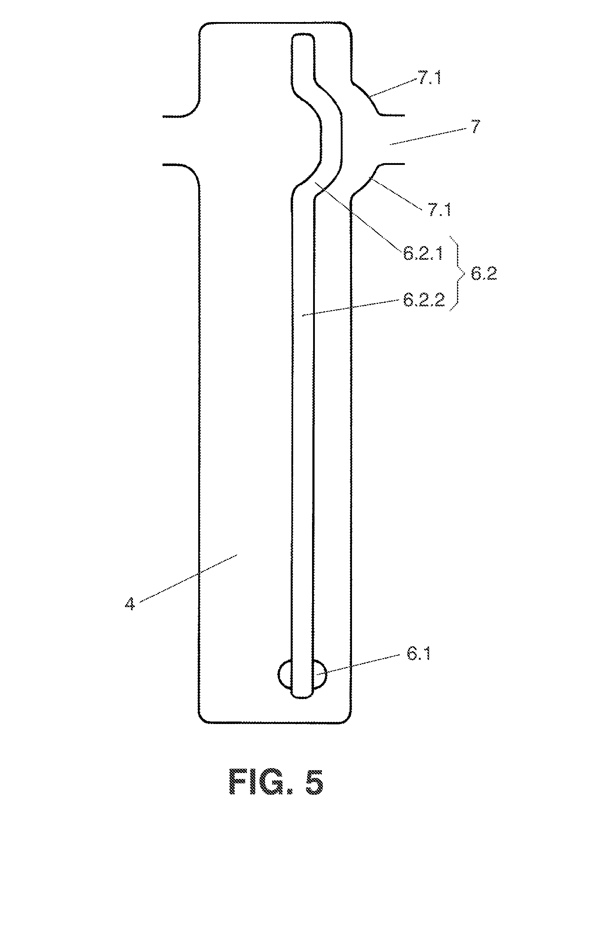

Figure 5 shows an embodiment of a cantilever (6.2) arranged in a second cavity (4), with the peculiarity that the first sector (6.2.1) of the cantilever (6) has two concave-curved portions corresponding with two curved seats (7.1) in the opening (7) on which the cantilever (6.2) rests in order to close the opening in a valve closed position. This double seat improves the closure of the opening (7), preventing possible seepages.

In a preferred embodiment of the impulsion core of the invention, the cantilevers (6.2, 8.2) are sized in relation to the cavities (4, 5) in which they are arranged such that in a valve open position, there is a clearance between the edge of the cantilever (6.2, 8.2) and the cover which covers the cavities (4, 5), such that the fluid can circulate between the cantilever (6.2, 8.2) and the cover. In this case, a gasket can be provided in the valve seat to seal the gap existing between the cantilever (6.2, 8.2) and the cover when the valve (6, 8) is located in a closed position, such that the sealing is assured in reverse flow conditions. The cover can be the membrane (14) itself in the event that it covers the entire plate (2), or an additional cover in an embodiment in which the membrane (14) only covers the first cavity (3).

The pneumatic actuation of the membrane (14) can be achieved by means of the connection to an electro-pneumatic controller.

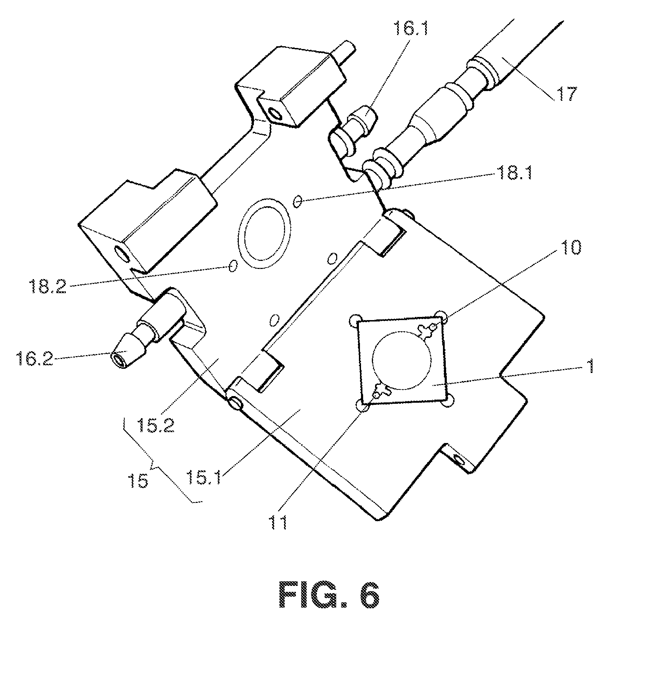

Figure 6 shows a fluid micropump comprising an impulsion core (1) and a casing (15) suitable for receiving the impulsion core (1). The impulsion core (1) housed in a first part (15.1) of the casing (15) is seen in the figure. In the embodiment of the figure, the casing (15) comprises two parts that are hinged together like a book. The first part (15.1) has retaining means for retaining the impulsion core. The second part (15.2) contains a pneumatic actuator suitable for causing the deformation and oscillation of the membrane (14) at a certain frequency. The impulse excitation of the membrane (14) is provided by means of a pneumatic duct (17) penetrating the second part (15.2). This mechanical excitation uses the flexibility of the membrane (14) of the impulsion core (1) to vary the volume of the first chamber (3) over time. The second part (15.2) further comprises connection adapters connected to an inlet duct (16.1) and outlet duct (16.2) for the pumped blood and fluid connection means (18.1, 18.2) suitable for connecting with the fluid inlet (10) and the fluid outlet (11) of the impulsion core (1). The casing (15) can be provided with fixing means for fixing the first part (15.1) of the casing (15) with respect to the second part (15.2) and/or with attachment joints assuring the leak-tightness with the impulsion core (1).

Advantageously, since the micropump is formed from a casing and an impulsion core that can be separated from one another, it is possible to dispose of the impulsion core, which is the component having a shorter service life, either because blood will be pumped from another individual or because one of its components has failed, whereas the casing can continue to be used.

Operating tests have been conducted on a micropump with an impulsion core such as that depicted in Figure 1, in this case, with an approximate size of 1 cm², with a volume in the first cavity of 15 µl, a height of 450 µm and a flow rate of 0.376 ml/min. In the embodiment of the example, the cantilevers (6.2, 8.2) are 1350 µm long, 250 µm high and 40 µm wide. The tests consist of passing a fluid with polystyrene particles about 6 µm in diameter in suspension at a concentration of 45% (v/v) through the micropump for 30'. Said concentration is within the hematocrit levels in humans. The amount of particles deposited around the cantilevers (6.2, 8.2) is low, which indicates a minimal probability of erythrocyte accumulation.

The applications of the impulsion core of the invention include:
- Research in clotting, hemofilia, thrombosis, platelet biology, tumor cell adhesion (long circulation times are required for low affinity), leukocyte adhesion, other aspects of leukocyte biology (those which benefit from flow conditions).
- Clinical or pharmacological research: materials for stents, anti-clotting drugs, drug tests in relation to side effects on clotting.
- Diagnosis: Detection of adherent tumor cells in the blood of a patient, clotting deficiencies.

The invention can be applied in parasitology studies, in which the circulation of the fluid sample can lead to better understanding of the biology and progression of infection, such as malaria.

In the case of studies with the malaria parasite *Plasmodium falciparum,* the impulsion core and the micropump of the invention can provide the basis for studying processes such as erythrocyte invasion, cytoadherence or rosette formation, as well as interactions between these processes, in closed circuit, continuous flow conditions imitating the physiological environment in which the parasite lives.

The asexual cycle of the malaria parasite P. *falciparum* in blood can be reproduced in culture conditions. This cycle lasts for 48 hours and starts with parasites invading an erythrocyte in the merozoite stage. Once the parasite is inside the erythrocyte, it goes through the ring, trophozoite and schizont stages, releasing new merozoites into circulation which in a few seconds will invade new erythrocytes. In standard invasion assays in static conditions, non-infected erythrocytes and schizonts are mixed together in static conditions, and between 12 and 20 hours later, the number of parasites in the ring stage are counted to quantify the invasion. The invasion of enzymatically treated erythrocytes, mutant erythrocytes, or the invasion in the presence of specific antibodies or inhibitors can be studied.

The erythrocytes infected with parasites in the trophozoite or schizont stage do not circulate freely, but rather they are adhered to the walls of the blood vessels (cytoadherence) through endothelial receptors. Furthermore, they can attach to non-infected erythrocytes (rosettes). It is suspected that the fact that a schizont is adhered to the walls of blood vessels or it is forming rosettes at the time the merozoites are released can affect the capacity of these merozoites to invade erythrocytes.

An object of the invention is the use of the impulsion core according to the invention to conduct cellular interaction assays to determine the cellular interaction of the cell components of the blood and, particularly, of the erythrocytes with P. *falciparum* in flow conditions, because the erythrocyte invasion process in physiological conditions occurs in flow conditions and the flow can have a critical effect on the process, particularly for the initial contact between the merozoite and the erythrocyte.

Said use would preferably be based on the following methodology:
(i) providing a closed circuit and an impulsion core according to the invention for impulsing the flow inside the closed circuit;
(ii) providing a fluid for filling the closed circuit;
(iii) providing erythrocytes and the parasite P. *falciparum* within the fluid; and
(iv) activating the impulsion core to circulate the fluid through the closed circuit.

The use according to the preceding methodology of the impulsion core according to the invention allows the development of P. *falciparum* in flow conditions which simulate the conditions in which the infections caused by this parasite occur.

To quantify the erythrocyte invasion by P. *falciparum,* an additional step of counting the number of parasites after a determined flow circulation time is included in the preceding methodology.

In the case of the study of the invasion in cytoadherence conditions, an additional step of the preceding method will comprise coating the walls of the closed circuit with endothelial receptors to simulate cytoadherence before providing the fluid in the closed circuit.

In the case of the study of the invasion in rosette formation conditions, in step (iii) of the described method, the parasite P. *falciparum* will be provided in the form of at least one strain capable of inducing the erythrocytes into rosette formation.

An alternative use relates to the study of the cytoadherence in the presence of the parasite P. *falciparum,* which would preferably be based on the following methodology:
i) providing a closed circuit and an impulsion core according to the invention for impulsing the flow inside the closed circuit;
(ii) coating the walls of the closed circuit with endothelial receptors to simulate cytoadherence before providing the fluid in the closed circuit;
(iii) providing a fluid for filling the closed circuit;
(iv) providing erythrocytes and the parasite P. *falciparum* in the form of at least one strain capable of inducing the infected erythrocytes into being cytoadherent within the fluid;
(v) activating the impulsion core to circulate the fluid through the closed circuit; and,
(vi) measuring the cytoadherence, preferably by means of microscopy.

An alternative use relates to the study of the rosette formation in the presence of the parasite P. *falciparum,* which would preferably be based on the following methodology:
(i) providing a closed circuit and an impulsion core according to the invention for impulsing the flow inside the closed circuit;
(ii) providing a fluid for filling the closed circuit;
(iii) providing erythrocytes and the parasite P. *falciparum* in the form of at least one strain capable of inducing the erythrocytes into rosette formation within the fluid;
(iv) activating the impulsion core to circulate the fluid through the closed circuit; and,
(v) detecting rosette formation, preferably by means of microscopy.

Other applications can include blood studies, cell cultures and drug tests. In particular, a miniaturized closed flow circuit with the flow impulsed by an impulsion core according to the invention can be applied in the diagnosis of clotting disorders or in drug tests targeting clotting or thrombosis, since these processes depend on the flow.

A diagnostic use for the diagnosis of clotting disorders or for drug tests targeting clotting or thrombosis would preferably be based on the following methodology:
(i) providing a closed circuit and an impulsion core according to the invention for impulsing the flow inside the closed circuit;
(ii) providing blood for filling the closed circuit;
(iii) activating the impulsion core to circulate the fluid through the closed circuit; and,
(vi) detecting the clot formation and/or platelet aggregation in the closed flow circuit.

The preceding use can include the addition of at least one compound inside the closed circuit.

The detection of clots and/or platelet aggregates can be performed in real time by means of monitoring with a microscope coupled to the circuit.

In another embodiment, the detection is performed in specific times by means of obtaining a sample from the closed circuit for subsequent analysis.

In both cases, the times and concentrations of the samples and/or compounds, if any, are optimized to prevent the circuit being blocked by large clots.

Another application is tumor cell detection in a biological fluid, preferably blood.

The circulating tumor cells (CTC) are cells which are detached from a primary tumor and can cause metastasis. Their detection and characterization are very valuable for predicting metastasis formation, but their scarcity makes them very difficult to detect.

An additional use of the impulsion core is the study of CTC in flow conditions, like the conditions in which they are physiologically found. Others aspects of their biology that can be studied are their adherence to different receptors in flow conditions.

A use of the impulsion core of the invention for tumor cell detection in a fluid could be based on the following methodology:
(i) providing a closed circuit and an impulsion core according to the invention for impulsing the flow inside the closed circuit;
(ii) providing a biological fluid, preferably blood, for filling the closed circuit;
(iii) coupling a sensor suitable for tumor cell detection to the closed circuit; and
(iv) activating the impulsion core to circulate the fluid through the closed circuit;
(v) determining the presence of tumor cells after a determined flow circulation time.

With a closed flow circuit like this one coupled to a sensor suitable for the detection in the patient's blood, sensitivity is increased with respect to other systems as a result of the passage of the CTC multiple times through the sensor.

Furthermore, the device can be used in basic research on many processes or diseases that are critically affected by the flow and which would benefit from using a flow circulating through a closed circuit in conditions favorable for cells, such as clotting, thrombosis, leukocyte adhesion, etc.

## Claims

1. An impulsion core (1) for a fluid micropump comprising:
● a plate (2) with a first cavity (3),
● a second cavity (4) communicated with the first cavity (3), this second cavity (4) having at least one one-way valve (6) formed by means of a support (6.1) and a cantilever (6.2) emerging from the support (6.1) being configured to act by bending and establish the closure, the cantilever (6.2) comprising a first sector (6.2.1) suitable for closing a first opening (7) in a valve closed position and a second sector (6.2.2) which is arranged between the support (6.1) and the first sector (6.2.1),
● a third cavity (5) communicated with the first cavity (3), this third cavity (5) having at least one one-way valve (8) also formed by means of a support (8.1) and a cantilever (8.2) emerging from the support (8.1) being configured to act by bending and establish the closure, the cantilever (8.2) comprising a first sector (8.2.1) suitable for closing a second opening (9) in a valve closed position and a second sector (8.2.2) which is arranged between the support (8.1) and the first sector (8.2.1),
wherein one of the one-way valves allows the entrance of fluid into the first cavity (3) and the other one-way valve allows the exit of fluid from the first cavity (3),
● a fluid inlet (10) from the outside of the plate (2) towards the third cavity (5), and a fluid outlet (11) from the second cavity (4) towards the outside of the plate (2),
● a flexible membrane (14) closing the first cavity (3), suitable for impulsing a fluid by means of applying a pressing force on said membrane (14) in an operative situation,
wherein:
● the support (6.1, 8.1) and the second sector (6.2.2, 8.2.2) of said cantilever (6.2, 8.2) are separated from the walls of the cavity (4, 5) in which is located the opening (7, 9) which the valve (6, 8) closes such that a communication channel through which a fluid can circulate between either side of the cantilever (6.2, 8.2) around the support (6.1, 8.1) is established.

2. The impulsion core (1) according to claim 1, **characterized in that** the valve seat for supporting at least one of the cantilevers (6.2, 8.2) for closing the corresponding opening (7, 9) has two seats that are concave-curved towards the cavity in the allowed flow direction.

3. The impulsion core (1) according to claim 2, wherein the first sector (6.2.1, 8.2.1) of at least one of the cantilevers (6.2, 8.2) has a double curved sector in correspondence with the double valve seat.

4. The impulsion core (1) according to any of the preceding claims, **characterized in that** the cavities are configured by means of slots giving rise to a planar base and walls perpendicular to the plate.

5. The impulsion core (1) according to any of the preceding claims, **characterized in that** it is manufactured in SU-8.

6. The impulsion core (1) according to any of the preceding claims, **characterized in that** the membrane (14) is manufactured in elastomer.

7. The impulsion core (1) according to any of the preceding claims, **characterized in that** at least one support (13) is provided in the first cavity (3) to prevent the membrane (14) from knocking against the bottom of the cavity (3).

8. The impulsion core (1) according to any of the preceding claims, **characterized in that** it comprises a stopper (12) limiting the motion range of the cantilever in the valve open direction.

9. A fluid micropump comprising:
● an impulsion core (1) according to any of claims 1 to 8,
● retaining means for retaining the impulsion core,
● application means for applying a pressing force, configured to act on the membrane (14) of the impulsion core (1),
● fluid connection means (18.1) suitable for connecting with the fluid inlet (10) of the impulsion core (1), and
● fluid connection means (18.2) suitable for connecting with the fluid outlet (11) of the impulsion core (1).

10. The fluid micropump according to claim 9, wherein:
● the retaining means for retaining the impulsion core are arranged in a first part (15.1) of a casing (15),
● the application means for applying a pressing force are arranged in a second part (15.2) of the casing (15), and comprising fixing means for fixing the first part (15.1) with respect to the second part (15.2).

11. Use of an impulsion core according to any of claims 1-8 for the study of biological processes under closed circuit, continuous flow conditions.

12. Use according to claim 11 for the study of a biological process relating to the cellular interaction with the parasite *Plasmodium falciparum.*

13. Use according to claim 12, wherein the biological process is one selected from: erythrocyte invasion, cytoadherence and rosette formation, or the interaction between at least two of them.

14. Use of an impulsion core according to any of claims 1-8 for the diagnosis of blood clotting disorders.

15. Use of an impulsion core according to any of claims 1-8 for tumor cell detection in a biological fluid.
